(19) Europäisches Patentamt
European Patent Office
Office européen des brevets

(11) Publication number: 0 000 073 A1

# (12) EUROPEAN PATENT APPLICATION

(21) Application number: 78100144.1

(22) Date of filing: 13.06.78

(51) Int. Cl.[2]: C 07 C 172/00, A 61 K 31/59, //C 07 J 9/00, C 07 J 71/00, C 07 J 11/00

(30) Priority: 13.06.77 US 805677

(43) Date of publication of application: 20.12.78 Bulletin 78/1

(84) Designated Contracting States: BE CH DE FR GB LU NL SE

(71) Applicant: Merck & Co., Inc.,
Patent Department 126 East Lincoln Avenue,
Rahway, N.J. 07065 (US)

(72) Inventor: Jones, Howard,
5 Mountain Lane,
Holmdel, N.J. 07733 (US)

(72) Inventor: Yang, Shu Shu,
14 Wildwood Drive,
Piscataway, N.J. 08854 (US)

(72) Inventor: Dorn, Conrad Peter, Jr.,
972 Fernwood Avenue,
Plainfield, N.J. 07062 (US)

(74) Representative: Abitz, Walter, Dr.-Ing. et al,
Abitz, Morf, Gritschneder P.O. Box 860109,
D-8000 München 86 (DE)

## (54) 24-Dehydrovitamin D3 derivatives and preparation thereof and compositions containing same.

(57) 24-Dehydrovitamin $D_3$ derivatives, protected against metabolic conversions at the 25-position, of the formula

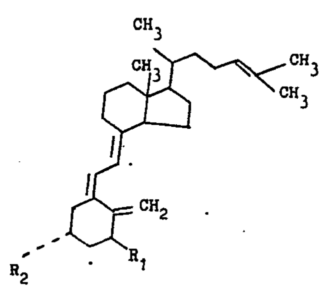

wherein $R_1$ is a member selected from the group consisting of hydrogen and hydroxy; $R_2$ is a member selected from the group consisting of hydrogen, hydroxy and fluoro, and wherein $R_1$ is not hydroxy when $R_2$ is fluoro, their preparation, pharmaceutical compositions, methods of promoting intestinal calcium transport and treating rickets, osteomalacia, and osteoporosis, including steroid induced osteoporosis, senile osteoporosis and post-menopausal osteoporosis, intermediates and their preparation are disclosed.

EP 0 000 073 A1

Croydon Printing Company Ltd.

# "24-DEHYDROVITAMIN $D_3$ DERIVATIVES AND PREPARATION THEREOF AND COMPOSITIONS CONTAINING SAME"

## BACKGROUND OF THE INVENTION

Known vitamin D compounds, such as cholecalciferol derivatives and metabolites thereof, promote both intestinal-calcium and phosphate transport and in conjunction with parathormone promote bone-calcium mobilization (bone resorbtion).

It is an object of the invention to find a class of novel vitamin $D_3$ derivatives which promote intestinal-calcium transport without the usual magnitude of bone-calcium mobilization, i.e., selectively promote intestinal transport, as opposed to bone mobilization. This differential effect has been widely sought for treatment of osteoporosis, especially that induced by an insufficient amount of calcium in relationship to the amount of phosphate.

## DETAILED DESCRIPTION OF THE INVENTION

In its composition aspect, the instant invention may be described as residing in the concept

of a new and useful group of vitamin $D_3$ derivatives classifiable in the field of organic chemistry as 24-dehydrovitamin $D_3$ derivatives. The novel 24-dehydrovitamin $D_3$ derviatives of the instant invention are members selected from the group consisting of 24-dehydrovitamin $D_3$, 24-dehydro-1α- hydroxy-vitamin $D_3$, 24-deydro-3β-desoxy-1α hydroxy-vitamin $D_3$ and 24-dehydro-3β-desoxy-3β-fluoro-vitamin $D_3$. These novel compounds may be represented by the following structural formula:

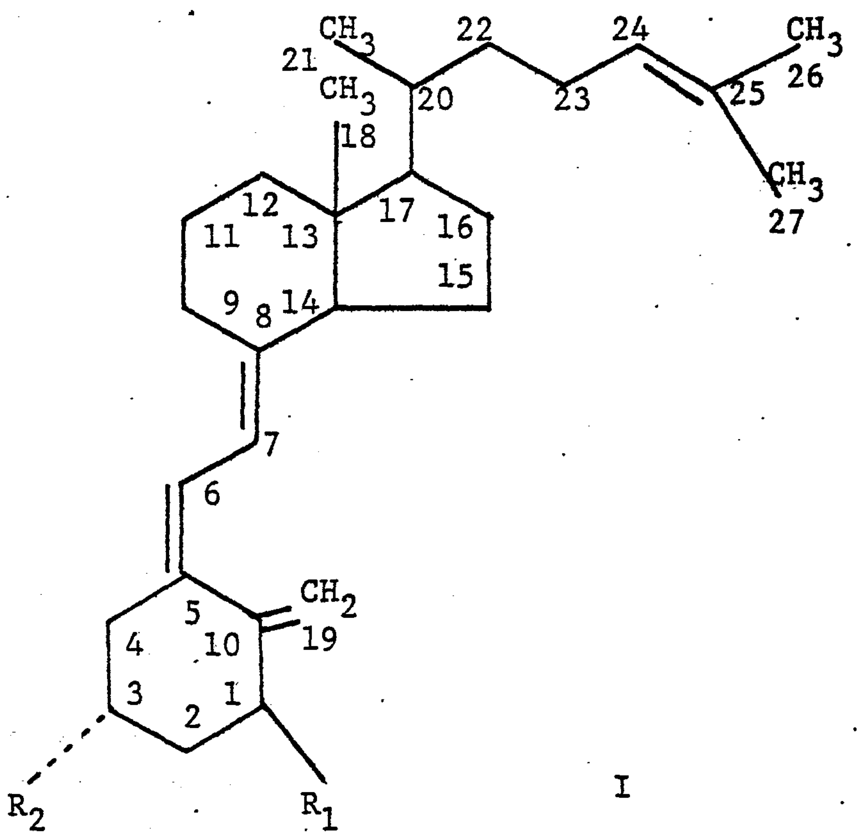

I

wherein $R_1$ is a member selected from the group consisting of hydrogen and hydroxy; $R_2$ is a member selected from the group consisting of hydrogen, hydroxy and fluoro; and wherein $R_1$ is not hydroxy when $R_2$

is fluoro.

The instant invention is based upon applicants' discovery that vitamin $D_3$ and derivatives may be metabolically blocked by the presence of a double bond at position $C_{24}$-$C_{25}$. The presence of such double bond retards metabolic hydroxylation in vivo in the 25-position. These novel 24-dehydrovitamin $D_3$ derivatives promote intestinal-calcium transport as opposed to bone-calcium mobilization. It is contemplated, therefore, that pharmaceutically acceptable dosage units containing a therapeutically effective quantity of the novel 24-dehydrovitamin $D_3$ derivatives of this invention will be administered orally or parenterally to patients, including humans and warm-blooded animals, in need of vitamin $D_3$ therapy to promote intestinal-calcium transport and in the treatment of rickets, osteomalacia, and osteoporosis including steroid-induced osteoporosis, senile osteoporosis and post-menopausal osteoporosis.

24-dehydrovitamin $D_3$ may be prepared readily by the photolysis of cholesta-5,7,24-trien-3β-ol (J. P. Moreau, D. J. Aberhart and E. Caspi, J. Org. Chem., Vol. 39, No. 14, pp. 2018, 1974) in order to obtain 24-dehydroprevitamin $D_3$. The irradiation conveniently is carried out at low temperatures, about 0 to 5°C, in a suitable organic solvent, such as deoxygenated ether, using a 450 watt Hanovia medium pressure mercury vapor lamp. Photolysis under these conditions usually is carried out for as short as 2 minutes or up to about 1 hour but usually is in the range of 4 to 10 minutes while

the reaction mixture is under a blanket of nitrogen. The photolysate usually will contain a mixture of vitamin and previtamin together with some starting material and/or other isomeric forms. The desired previtamin, however, usually is the predominating product and can be separated and purified by low temperature preparative thin layer chromatography on silica gel plates (1000 or 2000μ) at about 5°C developed with a suitable organic solvent or solvent mixture such as, for example, 10 to 15% acetone in hexane. Desirably the purification is carried out in the dark.

The purified 24-dehydroprevitamin $D_3$ then is dissolved in a suitable organic solvent such as deoxygenated ethanol and heated at relux for 1 to 2 hours. The desired 24-dehydrovitamin $D_3$ is separated and purified by low temperature preparative thin layer chromatography as described above.

If desired, the starting cholesta-5,7,24-trien-3β-ol may be prepared from the known cholesta 5,7,-dien-3β,25-diol-3-acetate by first treating this diene in a suitable organic solvent such as methylene chloride, chloroform, carbon tetrachloride, ethyl acetate and the like with 4-phenyl-1,2,4-triazoline-3,5-dione in order to prepare the 4-phenyl-1,2,4-triazoline-3,5-dione adduct and to protect the cholesta 5,7-diene system during subsequent dehydration. The cholesta-5,7-dien-3β-25-diol-3-acetate adduct in a suitable organic solvent such as benzene, hexane, xylene and the like, then is treated at about 25° to 40°C with a dehydrating agent such as phosphorous pentoxide in order to

obtain the 4-phenyl-1,2,4-triazoline-3,5-dione adduct of cholesta-5,7,24-trien-3β-ol-3-acetate (applicants have found that the 5,7,24-triene only is formed by this dehydration technique without formation of the corresponding 5,7,25-triene isomer) which then is treated with lithium aluminum hydride in dry tetrahydrofuran or other suitable solvent such as lower alkyl ether to remove the triazoline-dione group and obtain the desired cholesta-5,7,24-trien-3β-ol. This reaction, conveniently, is carried out at the reflux temperature of the solvent under an inert atmosphere (nitrogen or the like) and usually requires about 1 to 2 hours for completion.

24-dehydro-1α-hydroxyvitamin $D_3$ may be prepared from the known cholesta-5,7-dien-1α,3β,25-triol-1,3-diacetate by treating this compound with a dehydrating agent such as methyl (carboxysulfamoyl) triethylammonium hydroxide inner salt in dry benzene or other suitable organic solvent such as toluene or xylene in order to obtain cholesta-5,7,24-trien-1α,3β-diol-1,3-diacetate together with the corresponding 5,7,25-triene. The reaction conveniently is carried out at the reflux temperature of the solvent under an inert atmosphere and usually requires about 20 to 60 minutes for completion. Removal of the solvent and purification by conventional preparative thin layer chromatography gives the desired 5,7,24-trien-1,3-diacetate which may be irradiated and isomerized to obtain 24-dehydro-1α-hydroxyprevitamin $D_3$ diacetate and 24-dehydro-1α-hydroxyvitamin $D_3$ diacetate, respectively, by the techniques already discussed above. Conventional basic hydroylsis of the 24-

dehydro-1α-hydroxyvitamin $D_3$ diacetate using, for example, an alkali metal hydroxide such as 2.5 N aqueous sodium hydroxide gives 24-dehydro-1α-hydroxyvitamin $D_3$ which may be purified by conventional low temperature preparative thin layer chromatography. If desired, the 24-dehydro-1α-hydroxyprevitamin $D_3$ may be hydrolyzed prior to the irradiation step.

24-dehydro-3β-desoxy-3β-fluoro-vitamin $D_3$ may be prepared from the known 25-hydroxycholesterol dibenzoate by first selectively hydrolyzing this compound at the 3-position with an alkali metal hydroxide such as 2.5 N aqueous sodium hydroxide to give 25-benzoyloxycholesterol. The reaction conveniently is carried out in a suitable solvent such as tetrahydrofuran, ethanol or mixtures thereof under an inert atmosphere. Completion of the reaction requires 36 to 60 hours at room temperature. Removal of the solvent gives the crude product which is purified by conventional recrystallization.

The 25-benzoyloxycholesterol then is treated with a fluorinating agent such as diethylaminosulfur trifluoride in a suitable organic solvent such as methylene chloride, chloroform, ethyl acetate and the like at low temperatures ranging from about -80 to -60°C under an inert atmosphere. Upon warming to room temperature and standing for about an hour the crude product, 25-benzoyloxy-3β-fluorocholesta-5-ene, is separated and purified by chromatography on silica gel eluting with methylene chloride or the like.

The 25-benzoyloxy-3β-fluorocholest-5-ene then is treated with a brominating agent such as dibromodimethylhydantoin in boiling hexane under nitrogen and refluxed for about 30 minutes to give 25-benzoyloxy-3β-fluoro-7-bromocholest-5-ene which is dissolved in a suitable organic solvent such as xylene and added to a refluxing solution of trimethylphosphite in the same solvent also under nitrogen. The mixture is refluxed for about an hour and concentrated to give a crude mixture of 25-benzoyloxy-3β-fluorocholest-5,7-diene along with the corresponding 4,6-diene. This diene mixture then may be treated with 4-phenyl-1,2,4-triazoline-3,5-dione as previously described in order to obtain cyclo adducts. Chromatography on silica gel eluting with methylene chloride gives the desired 4-phenyl-1,2,4-triazoline-3,5-dione adduct of 25-benzoyloxy-3β-fluoro-cholesta-5,7-diene.

The above cholesta-5,7-diene cyclo adduct then is converted to 3β-fluorocholesta-5,7-dien-25-ol by treatment with lithium aluminum hydride in a refluxing organic solvent such as tetrahydrofuran. The mixture is refluxed for 30 to 90 minutes desirably in the dark and under an inert atmosphere. The 3β-fluorocholesta-5,7-dien-25-ol so produced then is dehydrated with methyl(carboxysulfamoyl)triethylammonium hydroxide inner salt as previously described to obtain 3β-fluorocholesta-5,7,24-triene which is subjected to irradiation and isomerization as described above to obtain, respectively, 24-dehydro-3β-desoxy-3β-fluoro-vitamin $D_3$ and 24-dehydro-3β-desoxy-3β-fluoro vitamin $D_3$.

24-dehydro-3β-desocy-1α-hydroxyvitamin $D_3$ may be prepared from 1α,25-dihydroxycholesta-5,7-diene (W. H. Okamura, M. N. Mitra, D. A. Procsal and

A. W. Norman, Biochem. and Biophys. Res. Comm., Vol. 65, No. 1, pp 24, 1975) by first selectively acetylating this compound at the 1-position with an acetylating agent such as acetic anhydride. The reaction conveniently is run in an organic solvent such as pyridine initially at a temperature between 0° and 5°C. After addition of the acetic anhydride is complete the reaction mixture is allowed to warm to room temperature and stirred for an additional 4 to 6 hours. The crude 1α-acetoxy-25-hydroxycholest-5,7-diene is separated and purified by conventional chromatography on silica gel eluting with chloroform. This product then may be dehydrated by treatment with methyl(carboxysulfamoyl)triethyl ammonium hydroxide inner salt by techniques previously described to obtain 1α-acetoxy-cholest-5,7,24-triene together with the corresponding 5,7,25-triene. The mixture is separated by conventional preparative thin layer chromatography employing, for example, 10% silver nitrate impregnated silica gel and developing with 10% acetone in hexane to obtain the pure 5,7,24-triene.

The acetoxy group at the 1-position then may be removed by conventional basic hydrolysis employing an alkali metal hydroxide such as 2.5 N aqueous sodium hydroxide to obtain 1α-hydroxycholesta-5,7,24-triene which then may be irradiated and isomerized by techniques described above to obtain, respectively, 24-dehydro-3β-desoxy-1α-hydroxy-previtamin $D_3$ and 24-dehydro-3β-desoxy-1α-hydroxyvitamin $D_3$.

As pointed out above, another aspect of the

instant invention relates to novel pharmaceutical compositions for promoting intestinal-calcium transport and for treating osteoporosis comprising a therapeutically effective quantity of the 24-dehydrovitamin $D_3$ derivatives described above as the essential active ingredient together with a non-toxic pharmaceutically acceptable carrier. Such compositions also may be used in the treatment of secondary hyperparathyroidis,, especially that induced by an insufficient amount of calcium in relationship to the amount of phosphate.

The non-toxic pharmaceutical carrier may be, for example, either a solid or liquid. Exemplary of solid carriers are lactose, corn starch, gelatin, talc, sterotix, stearic acid, magnesium stearate, terra alba, sucrose, agar, pectin and acacia. Exemplary of liquid carriers are peanut oil, olive oil, sesame oil and water. Similarly, the carrier or diluent may include a time delay material such as glyceryl monostearate or glyceryl distearate, alone, or with a wax.

The treatment of osteoporosis and secondary hyperparathyroidism in accordance with the method of the present invention is accomplished by orally or parenterally administering to patients a compound of formula I supra, or mixtures thereof in a non-toxic pharmaceutically acceptable carrier.

Several pharmaceutical forms of the therapeutically useful compositions may be used. For example, if a solid carrier is used, the compositions may take the form of tablets, capsules, powders,

troches or lozenges, prepared by standard pharmaceutical techniques. If a liquid carrier is used, the preparation may be in the form of a soft gelatin capsule, a syrup, a liquid solution, a liquid emulsion or a liquid suspension.

The active compounds of formula I, supra, are administered in a therapeutically effective amount sufficient to treat osteoporosis and secondary hyperparathyroidis,. The metabolically blocked 24-dehydrovitamin $D_3$ derivatives will reduce the bone mobilization in those cases wherein clinical symptoms have not been observed, e.g., administered prophylactically to persons subject to steroid induced osteoporosis, and in addition retard bone mobilization in those cases wherein clinical symptoms have been observed, e.g., senile osteoporosis, post-menopausal osteoporosis and secondary hyperparathyroidism. Advantageously, the active compounds of formula I, supra, will be administered alone or in a pharmaceutical composition in an amount of from about 1.0 to 3000 International Units (IU) per day, preferably from about 10 to 500 IU/day. Standard preparations of vitamin $D_3$ have an activity of about 40 IU/$\mu$g. The daily dosage may be given either in single or multiple dosages.

The method of treatment of this invention comprises administering to a patient (warm-blooded animal or human) the compound of formula I, supra, as previously described admixed with a non-toxic pharmaceutical carrier such as exemplified above. It should be understood that although preferred dosage ranges are given, the dose level for any

particular patient depends upon the activity of the specific compound employed. Also, many other factors that modify the actions of drugs will be taken into account by those skilled in the art in the therapeutical use of medicinal agents, particularly those described above; for example, body weight, sex, diet, time of administration, route of administration, rate of excretion, drug combination, reaction sensitivities and severity of the particular disease.

The best mode contemplated by applicants for carrying out their invention is set forth in the following examples, no limitation, however, being intended except as set forth in the appended claims.

## EXAMPLE 1

### 24-dehydrovitamin $D_3$

Step A: 4-phenyl-1,2,4-triazoline-3,5-dione Adduct of Cholesta-5,7-dien-3β,25-diol-3-acetate

To a solution of cholesta-5,7-dien-3β,25-diol-3-acetate (1.15 gm) in methylene chloride (30 ml), is added 4-phenyl-1,2,4-triazoline-3,5-dione (0.49 gm) in portions. After the addition is completed, the reaction mixture is concentrated to dryness and the residue is triturated with ether (about 15 ml). The crystalline mixture is aged at 0-5°C for 1 hour and then filtered. The crystalls are redisolved in methylene chloride and evaporated to dryness. The ether trituration is repeated to give the title product (1.55 gm, m.p. 160.5-162.5°C).

Step B: 4-phenyl-1,2,4-triazoline-3,5-dione Adduct of Cholesta-5,7,24-trien-3β-ol-3-acetate

To a stirred solution of the cholesta-5,7-dien-3β,25-diol-3-acetate cyclo adduct of Step A (1.50 g) in dry benzene (320 ml) at 30-35°C, is added phosphorous pentoxide (about 5 gm) in portions. After the dehydration is complete, the supervatant solution is decanted onto cold sodium bicarbonate solution. The organic layer is separated, washed with cold water, brine and dried. Removal of the solvent affords the crude product which is purified by chromatography on silica gel (60 gm). Elution with chloroform followed by elution with 1% acetone in chloroform gives the title product (1.18 gm, glass).

Step C: Cholesta-5,7-24-trien-3β-ol

To a stirred suspension of lithium aluminum hydride (1.8 gm) in dry tetrahydrofuran (200 ml), is added slowly a solution of the 4-phenyl-1,2,4-triazoline-3,5-dione adduct of cholesta-5,7-24-trien-3β-ol-3-acetate (1.0 gm) obtained in Step B in tetrahydrofuran (100 ml). The mixture is heated to reflux under nitrogen for 1.25 hours. The mixture is cooled in an ice-bath and quenched with saturated aqueous sodium sulfate solution (about 150 ml). The organic layer is separated and the aqueous layer is extracted once with tetrahydrofuran (200 ml). The combined tetrahydrofuran solution is concentrated *in vacuo* and extracted with chloroform (200 ml). The chloroform solution is washed with water, brine and dried. The crude

product is purified by low temperature preparative thin layer chromatography on silica gel plates (2000μ) at 5°C in the dark and developed with 6.5% acetone in chloroform. The main zone is extracted with 5% methanol in chloroform in the cold. Removal of the solvent followed by recrystallization from methanol yields the title product (503 mg, m.p. 104-107°C).

Step D: 24-dehydroprevitamin $D_3$

Cholesta-5,7,24-trien-3β-ol(330 mg) in deoxygenated ether (550 ml) is irradiated in a quartz photocell at 0 to 5°C for 9 minutes using a 450 watt Hanovia medium pressure mercury vapor lamp while a stream of nitrogen is bubbled through the solution. Thin layer chromatography on 5% silver nitrate impregnated silica gel (developed with 14% acetone in hexane) shows formation of a mixture containing 24-dehydrolumisterol$_3$ (Rf 0.12), starting material (Rf 0.37), 24-dehydrotachysterol$_3$ (Rf. 0.50) and 24-dehydroprevitamin $D_3$ (Rf 0.57). The photo irradiated gross mixture is purified by low temperature preparative thin layer chromatography on silica gel plates (2000μ) at 5°C developed with 15-16% acetone in hexane. The main zone is concentrated to a residue to obtain the title product (90 mg).

Step E: 24-dehydrovitamin $D_3$

24-dehydroprevitamin $D_3$ (80 mg) from Step D is dissolved in deoxygenated absolute ethanol (5 ml) and heated under reflux for 2 hours under a nitrogen

atmosphere. The mixture is concentrated in vacuo to a residue. Thin layer chromatography on 5% silver nitrate impregnated silica gel developed with 20% acetone in hexane indicates the presence of 24-dehydrolumisterol$_3$ (Rf 0.18), 24-dehydrovitamin $D_3$ (Rf 0.46) and 24-dehydroprevitamin $D_3$ (Rf 0.58) in the approximate proportions of 1:6:2. Purification of the mixture by low temperature preparative thin layer chromatography on silica gel plates (1000$\mu$) developed in 16% acetone in hexane at 5°C in the dark yields the title product (60 mg, glass): nmr ($CDCl_3$)$\delta$ 0.55 (3H, s , 18-Me), 0.93 (3H, d, J = 4.5 Hz, 21-Me), 1.59 (3H, s, 26-Me), 167 (3H, S, 27 Me), 3.9 (1H, m, C-3-H), 4.80 and 5.03 (3H, two $AB_q$ and one hidden tripplet, =$CH_2$ and C-24-H) and 5.97 and 6.23 ppm (2H, $AB_q$, J=11.5Hz, C-6,7H's).

## EXAMPLE 2

### 24-dehydro-1α-hydroxyvitamin $D_3$

Step A: Cholesta-5,7,24-trien-1α,3β-diol-1,3-diacetate

A mixture of cholesta-5,7-dien-1α,3β-25-triol-1,3-diacetate (350 mg) and methyl (carboxysufamoyl)triethylammonium hydroxide inner salt (350 mg) in dry benzene (30 ml) is heated to reflux under nitrogen for 1/2 hour. After cooling, the reaction mixture is quenched with water (10 ml). The organic phase is separated, washed, dried and concentrated. The crude product contains both cholesta-5,7,24-trien-1α,3β-diol-1,3-diacetate and cholesta-5,7,25-trien-1α,3βdiol-1,3-diacetate.

The crude products are separated by preparative thin layer chromatography on 10% silver nitrate impregnated silica gel developed with 15% acetone in hexane.

Step B: 24-dehydro-1α-hydroxyprevitamin $D_3$ Diacetate

Cholesta-5,7,24-trien-1α-3β-diol-1,3-diacetate (80 mg) in deoxygenated ether (500 ml) is irradiated in a quartz photocell at 0 to 5°C for 5 minutes using a 450 watt Hanovia medium pressure mercury vapor lamp while a stream of nitrogen is bubbled through the solution. The photolysis mixture is purified by low temperature preparative thin layer chromatography in the dark on silica gel plates (2000μ) at 5°C developed with 15% acetone in hexane to obtain the title product.

Step C: 24-dehydro-1α-hydroxyvitamin $D_3$

The purified 24-dehydro-1α-hydroxyprevitamin $D_3$ diacetate of Step B is dissolved in deoxygenated absolute alcohol and heated under reflux for 2 hours. The resulting solution containing a mixture of vitamin and previtamin is added to an equal volume of tetrahydrofuran and ethanol and one-fifth volume of 2.5 N sodium hydroxide solution under nitrogen at room temperature and allowed to stand overnight. Low temperature preparative thin layer chromatography of the hydrolysis mixture in the dark on silica gel plates (1000μ) at 5°C developed with 15% acetone in chloroform gives the title product.

EXAMPLE 3

24-dehydro-3β-desoxy-3β-fluorovitamin $D_3$

Step A: 25-benzoyloxycholesterol

To a stirred solution of 25-hydroxycholesterol dibenzoate (4.9 gm) in tetrahydrofuran (420 ml) and ethanol (210 ml) under nitrogen, is added 2.5 N sodium hydroxide (84 ml). The reaction mixture is stirred at room temperature under nitrogen for 48 hours and then acidified with glacial acetic acid. The mixture is concentrated *in vacuo* to about 100 ml, diluted with water (about 100 ml) and the precipitate is separated by filtration. The crystals are dissolved in chloroform (300 ml), washed with bicarbonate solution, brine and dried. Removal of the solvent yields the crude product which is recrystallized from methanol (3.23 gm, m.p. 134-135.5°C).

Step B: 25-benzoyloxy-3β-fluorocholest-3-ene

To a solution of diethylaminosulfur trifluoride (0.09 ml) in methylene chloride (15 ml) at -78°C under nitrogen, is added a solution of 25-benzoyloxycholesterol (200 mg) in methylene chloride (20 ml). The mixture is allowed to warm to room temperature and is stirred for one hour. The reaction mixture is quenched with cold sodium bicarbonate solution and the organic phase is washed, dried and concentrated to a residue. Chromatography of the crude product on silica gel and elution with 5% acetone in hexane gives the title product.

Step C: 4-phenyl-1,2,4-triazoline-3,5-dione Adduct of 25-Benzoyloxy-3β-fluorocholesta-5,7-diene

To a boiling solution of 25-benzoyloxy-3β-fluorocholest-5-ene (10 gm) in hexane (500 ml) under nitrogen, is added dibromodimethyl hydantoin (4 gm). The mixture is refluxed for 30 minutes and cooled to room temperature. The insoluble solid is filtered off and the filtrate is concentrated *in vacuo* to give the 7-bromo derivative which is dissolved in xylene (90 ml). This solution is then added to a refluxing solution of trimethyl phosphite (25 gm) in xylene (35 ml) under nitrogen. The mixture is refluxed for a further 75 minutes and concentrated *in vacuo* to give crude 25-benzoyloxy-3β-fluorocholesta-5,7-diene along with 25-benzoyloxy-3β- fluorocholesta-4,6-diene. To a solution of the diene mixture in methylene chloride (70 ml), is added 4-phenyl-1,2,4-triazoline-3,5-dione (1.7 gm) in small portions. When the addition is complete, the mixture is then concentrated and the residue is chromatographed on silica gel. Elution with methylene chloride gives the title product.

Step D: 3β-fluorocholesta-5,7-dien-25-ol

A mixture of 4-phenyl-1,2,4-triazoline-3,5-dione adduct of 25-benzoyloxy-3β-fluorocholesta-5,7-diene (3 gm), tetrahydrofuran (450 ml) and lithium aluminum hydride (4 gm) is refluxed under nitrogen in the dark for 1 hour. The reaction mixture is cooled and is quenched with saturated

sodium sulfate solution. The organic phase is separated and concentrated to a residue. The wet residue is extracted with chloroform and the extract is concentrated to a residue which is chromatographed on silica gel and eluted with 10% acetone in chloroform to give the title product.

Step E: 3β-fluorocholesta-5,7,24-triene

A mixture of 3β-fluorocholesta-5,7-dien-25-ol (500 mg) and methyl(carbonxysulfamoyl)triethylammonium hydroxide inner salt (500 mg) in dry benzene (40 ml) is heated to reflux under nitrogen for 30 minutes. The cooled reaction mixture is quenched with water. The organic phase is separated, washed, dried and concentrated. The crude residue is purified by preparative thin layer chromatography on 10% silver nitrate impregnated silica gel developed with 10% acetone in hexane to give the title product.

Step F: 24-dehydro-3β-desoxy-3β-fluoroprevitamin $D_3$

3β-fluorocholesta-5,7,24-triene (100 mg) in deoxygenated ether (500 ml) is irradiated in a quartz photocell at 0 to 5°C for 6 minutes using a 450 watt Hanovia medium pressure mercury vapor lamp while a stream of nitrogen is bubbled through the solution. The photolysate is purified by low temperature preparative thin layer chromatography in the dark on silica gel plates (2000μ) developed with 10% acetone in hexane at 5°C to give the title product.

Step G: 24-dehydro-3β-desoxy-3β-fluorovitamin $D_3$

24-dehydro-3β-desoxy-3β-fluoroprevitamin $D_3$ (35 mg) is dissolved in deoxygenated absolute alcohol and heated to reflux under nitrogen for 2 hours. The resulting mixture of vitamin and previtamin is separated and purified by low temperature preparative thin layer chromatography in the dark on silica gel plates (2000μ) developed with 10% acetone in hexane at 5°C to give the title product.

EXAMPLE 4

24-dehydro-3β-desoxy-1α-hydroxyvitamin $D_3$

Step A: 1α-acetoxy-25-hydroxycholesta-5,7-diene

To a solution of 1α,25-dihydroxycholesta-5,7-diene (401 mg) in dry pyridine (10 ml) at 0 to 5°C, is added acetic anhydride (0.5 ml) dropwise. The mixture is stirred at room temperature for 6 hours, cooled, quenched with water and extracted with ethyl acetate. The organic phase is separated, washed, dried and concentrated to a residue. The crude residue is purified by chromatography on silica gel, eluted with chloroform, to give the title product.

Step B: 1α-acetoxycholesta-5,7,24-triene

A mixture of 1α-acetoxy-25-hydroxycholesta-5,7-diene (350 mg) and methyl(carboxysulfamoyl) triethylammonium hydroxide inner salt (350 mg) in

dry benzene (30 ml) is heated to reflux under nitrogen for 30 minutes. The mixture is cooled and quenched with water. The organic phase is separated, washed, dried and concentrated to a residue. The crude product containing 1α-acetoxycholesta-5,7,24-triene along with the corresponding 5,7,25-triene is purified by preparative thin layer chromatography on 10% silver nitrate impregnated silica gel developed with 10% acetone in hexane to give the title product.

Step C: 1α-hydroxycholesta-5,7,24-triene

A mixture of 1α-acetoxycholesta-5,7,24-triene (80 mg) tetrahydrofuran (2.0 ml), ethanol 2.0 ml), and 2.5 N sodium hydroxide (0.5 ml) is stirred at room temperature under nitrogen overnight. The reaction mixture is purified by low temperature preparative thin layer chromatography in the dark on silica gel plates (2000μ) developed with 15% acetone in hexane at 5°C to give the title product.

Step D: 24-dehydro-3β-desoxy-1α-hydroxyprevitamin $D_3$

1α-hydroxycholesta-5,7,24-triene (50 mg) in deoxygenated ether (500 ml) is irradiated at 0 to 5°C under nitrogen for 4 minutes using a 450 watt medium pressure Hanovia mercury vapor lamp. The photolysate is purified by low temperature preparative thin layer chromatography in the dark on silica gel plates (2000μ) at 5°C developed with 15% acetone in hexane to give the title product.

Step E: 24-dehydro-3β-desoxy-1α-hydroxyvitamin $D_3$

24-dehydro-3β-desoxy-1α-hydroxyprevitamin $D_3$ (40 mg) is dissolved in deoxygenated absolute ethanol and heated to reflux under nitrogen for 2 hours. The resulting mixture of vitamin and previtamin is purified by low temperature preparative thin layer chromatography in the dark on silica gel plates (2000μ) at 5% developed with 15% acetone in hexane to give the title product.

The subject matter which applicants' regard as their invention is particularly pointed out and distinctly claimed as follows:

1. A process for preparing a compound of the formula:

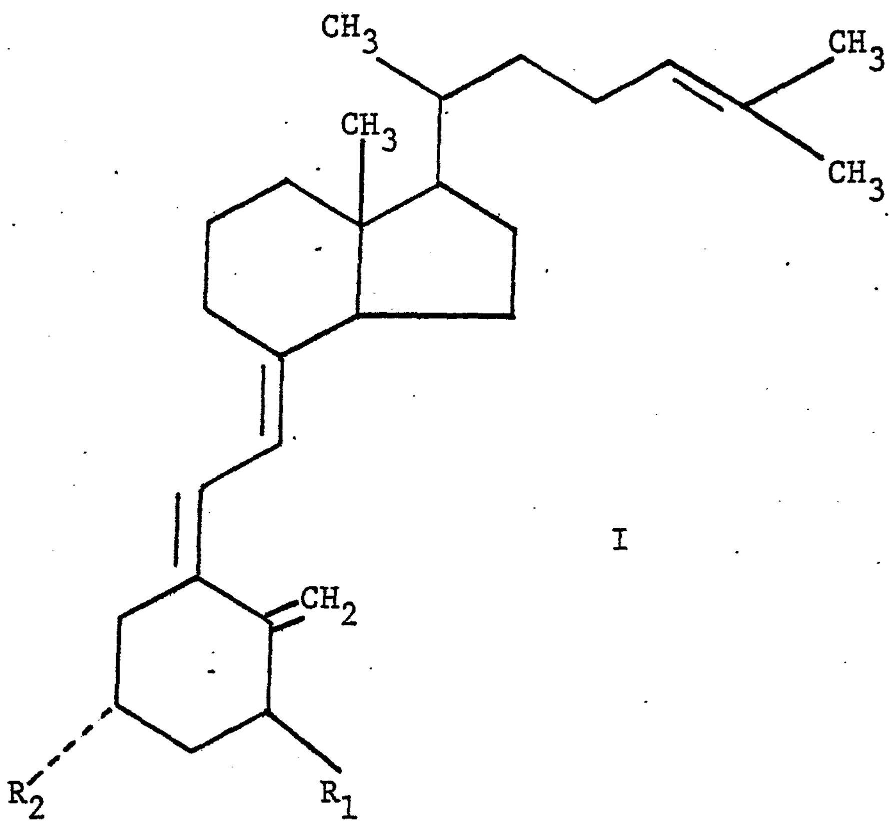

I

wherein $R_1$ is a member selected from the group consisting of hydrogen and hydroxy; $R_2$ is a member selected from the group consisting of hydrogen, hydroxy and fluoro; and wherein $R_1$ is not hydroxy when $R_2$ is fluoro, which comprises isomerizing a compound of the formula:

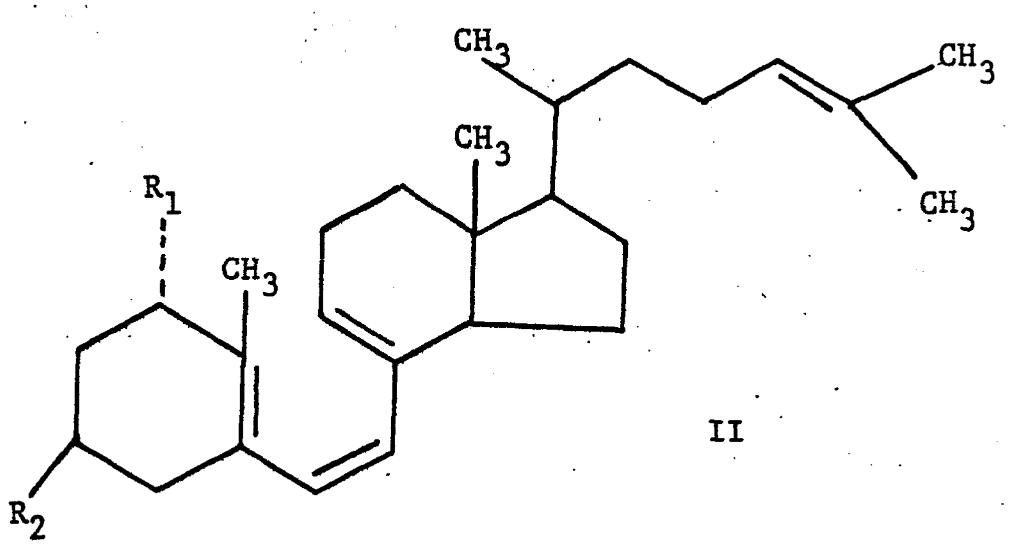

II

wherein $R_1$ is a member selected from the group consisting of hydrogen and hydroxy; $R_2$ is a member selected from the group consisting of hydrogen, hydroxy and fluoro; and wherein $R_1$ is not hydroxy when $R_2$ is fluoro, by heating in a refluxing organic solvent.

2. A compound selected from the group consisting of 24-dehydrovitamin $D_3$, 24-dehydro-1α-hydroxyvitamin $D_3$, 24-dehydro-3β-desoxy-1α-hydroxyvitamin $D_3$ and 24-dehydro-3β-desoxy-3β-fluorovitamin $D_3$.

3. The compound of Claim 2 which is 24-dehydrovitamin $D_3$.

4. The compound of Claim 2 which is 24-dehydro-1α-hydroxyvitamin $D_3$.

5. The compound of Claim 2 which is 24-dehydro-3β-desoxy-1α-hydroxyvitamin $D_3$.

6. The compound of Claim 2 which is 24-dehydro-3β-desoxy-3β-fluorovitamin $D_3$.

7. A pharmaceutical composition comprising a pharmaceutically acceptable carrier and a therapeutically effective quantity of a compound selected from the group consisting 24-dehydrovitamin $D_3$, 24-dehydro-1α-hydroxyvitamin $D_3$, 24-dehydro-3β-desoxy-1α-hydroxyvitamin $D_3$ and 24-dehydro-3β-desoxy-3β-fluorovitamin $D_3$.

European Patent Office

# EUROPEAN SEARCH REPORT

0000073

Application number
EP 78 10 0144

## DOCUMENTS CONSIDERED TO BE RELEVANT

| Category | Citation of document with indication, where appropriate, of relevant passages | Relevant to claim |
|---|---|---|
| X | TETRAHEDRON LETTERS, vol. 13, 1977 pages 1107-1108<br>* Whole article *<br>-- | 1-3,7 |
| A | FR - A - 2 162 113 (WESCONSIN ALUMNI)<br>* Claims 1-3 *<br>-- | 1,2,4, 7 |
| A | US - A - 3 906 014 (HECTOR F. DE LUCA)<br>* Claims 1,2; column 1, lines 20 to 25 *<br>-- | 1,2,5, 7 |
| A | CHEMICAL ABSTRACTS, vol. 82 (1975) 80397r & Ukr.Biokhim.Zh.1974 46(5) 627-30<br>* Abstract *<br>---- | 1,2,6, 7 |

CLASSIFICATION OF THE APPLICATION (Int. Cl.²)

C 07 C 172/00
A 61 K 31/59
// C 07 J 9/00
C 07 J 71/00
C 07 J 11/00

TECHNICAL FIELDS SEARCHED (Int.Cl.²)

C 07 C 172/00
A 61 K 31/59

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

✓ The present search report has been drawn up for all claims

| Place of search | Date of completion of the search | Examiner |
|---|---|---|
| The Hague | 05-09-1978 | HENRY |